(19)

Europäisches Patentamt
European Patent Office
Office européen des brevets

(11) **EP 4 300 515 A1**

(12) **EUROPEAN PATENT APPLICATION**
published in accordance with Art. 153(4) EPC

(43) Date of publication:
**03.01.2024 Bulletin 2024/01**

(21) Application number: **21928175.5**

(22) Date of filing: **15.04.2021**

(51) International Patent Classification (IPC):
*G16H 50/50* (2018.01)      *G16H 30/40* (2018.01)
*G16H 50/70* (2018.01)      *G16H 50/20* (2018.01)
*G06N 20/00* (2019.01)      *G06N 3/08* (2023.01)
*G06T 5/00* (2006.01)       *G01N 33/483* (2006.01)

(52) Cooperative Patent Classification (CPC):
**G01N 33/483; G06N 3/08; G06N 20/00; G06T 5/00;
G16H 10/00; G16H 30/40; G16H 50/20;
G16H 50/50; G16H 50/70**

(86) International application number:
**PCT/KR2021/004727**

(87) International publication number:
**WO 2022/181879 (01.09.2022 Gazette 2022/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.02.2021   KR 20210024360**

(71) Applicant: **Samsung Life Public Welfare
Foundation
Seoul 04348 (KR)**

(72) Inventor: **KIM, Kyoung-Mee
Seoul 06264 (KR)**

(74) Representative: **Ostertag & Partner Patentanwälte
mbB
Azenbergstraße 35
70174 Stuttgart (DE)**

(54) **DEEP LEARNING MODEL-BASED TUMOR-STROMA RATIO PREDICTION METHOD AND ANALYSIS DEVICE**

(57)      A deep learning model-based tumor-stroma ratio prediction method comprises the steps in which an analysis device: receives an input of a first staining image of target tissue; generates a second staining image by inputting the first staining image into a trained deep learning model; generates a first binary image of the first staining image; generates a second binary image of the second staining image; and calculates the tumor-stroma ratio of the target tissue on the basis of the first binary image and the second binary image.

FIG. 1

**Description**

[Technical Field]

The following description relates to a technique for predicting a tumor-stroma ratio (TSR) of cancer tissue using a deep learning model.

[Background Art]

A tumor-stroma ratio (TSR) is a ratio of tumor-related stroma in cancer tissue. It has proven that a high TSR is a predictor of bad prognosis in a plurality of tumors. In other words, accurate evaluation of a TSR is important for tumor diagnosis, prognosis prediction, and treatment.

[Disclosure]

[Technical Problem]

Conventionally, pathologists analyze tissue samples of patients to estimate tumor-stroma ratios (TSRs). This method depends on subjective judgement and thus does not ensure accuracy. Further, a method of quantifying stroma using a computer has appeared. However, this method only helps medical workers with their judgement and is not a technique for accurately assessing a TSR.

The following description provides a technique for accurately calculating a TSR by analyzing a patient's tissue using a deep learning model.

[Technical Solution]

In one general aspect, there is provided a method of predicting a tumor-stroma ratio (TSR) on the basis of a deep learning model, the method including receiving, by an analysis device, a first stained image of target tissue, generating, by the analysis device, a second stained image by inputting the first stained image into a trained deep learning model, generating, by the analysis device, a first binary image of the first stained image, generating, by the analysis device, a second binary image of the second stained image, and calculating, by the analysis device, a TSR of the target tissue on the basis of the first binary image and the second binary image.

In another aspect, there is provided an analysis device for predicting a TSR, the analysis device including an input device configured to receive a first stained image of target tissue, a storage device configured to store a generative adversarial model for generating a second stained image on the basis of the first stained image, and a calculation device configured to generate a first binary image by binzarizing the received first stained image, generate a virtual second binary image by inputting the received first stained image to the generative adversarial model, generate a second binary image by binarizing the virtual second stained image, and calculate a TSR of the target tissue on the basis of the first binary image and the second binary image.

[Advantageous Effects]

According to the following description a tumor-stroma ratio (TSR) is automatically calculated based on only one stained image of a patient's tissue. The following description provides a TSR which is objectively and highly accurate in a short time, thereby contributing to tumor diagnosis and treatment.

[Description of Drawings]

FIG. 1 illustrates an example of a system for predicting a tumor-stroma ratio (TSR);
FIG. 2 illustrates an example of a process of predicting a TSR;
FIG. 3 illustrates an example of a generative adversarial model;
FIG. 4 illustrates an example of a schematic process of training a generative adversarial model;
FIG. 5 illustrates an example of a process of aligning a haematoxylin and eosin (H&E) image and a cytokeratin (CK) image;
FIG. 6 illustrates an example of a process of training a generative adversarial model using an H&E image and a CK image;

FIG. 7 illustrates an example of an analysis device;

FIG. 8 is an example of a virtual CK image generated by a generative adversarial model; and

FIG. 9 is an example of evaluating accuracy of TSR prediction results based on a deep learning model.

[Modes of the Invention]

Since the following description may be diversely modified and have various embodiments, specific embodiments will be shown in the drawings and described in detail. However, it should be understood that this is not intended to limit the following description to specific embodiments, but to encompass all modifications, equivalents, and substitutions included in the spirit and scope of the following description.

Terms such as first, second, A, B, and the like may be used to describe various components, but the components are not limited by the above terms. The terms are used only to distinguish one component from other components. For example, without departing from the spirit of the following description, a first component may be referred to as a second component, and similarly, a second component may be referred to as a first component. The term "and/or" includes a combination of a plurality of associated listed items or any one of the plurality of associated listed items.

Among terms used herein, the singular expressions may include plural expressions, unless the context clearly dictates otherwise. It is to be understood that the term such as "include" and the like indicates the presence of described features, numerals, steps, operations, components, parts, or a combination thereof and does not preclude the presence or addition of one or more other features, numerals, steps, operations, components, parts, or a combination thereof.

Prior to a detailed description of drawings, it is to be noted that components herein are just distinguished by the main function of each component. In other words, two or more components to be described below may be combined into one component, or one component may be divided into two or more by subdivided function and provided. Also, each of the components to be described below may additionally perform some or all functions that are handled by other components in addition to main functions that the component is responsible for, and some main functions handled by each component may be exclusively performed by other components.

Further, in performing a method or an operating method, steps constituting the method may be performed in a different order from a described order unless a specific order is clearly described in the context. In other words, the steps may be performed in a described order, substantially simultaneously, or in the reverse order.

The following description is a technology for predicting a tumor-stroma ratio (TSR) of a specific sample. Conventionally, stained tissue images are used for TSR analysis.

The following description is an example of using two different types of stained images. As a first stained image, a haematoxylin and eosin (H&E) image is used. As a second stained image, cytokeratin (CK) image is used. The following description is not limited to a specific staining method, and any staining method in which tissue (stroma) is identified by one type of staining and tumors are identified by another type of staining may be used. The following description may be a method of predicting a TSR by automatically generating a second stained image on the basis of a specific first stained image. However, for convenience of description, it is assumed that a first stained image is an H&E image and a second stained image is a CK image.

The following description is related with generating a second stained image from a first stained image using a learning model. The learning model is a machine learning model. The learning model is one of various types of models. For example, the learning model is a decision tree model, a random forest model, a k-nearest neighbor (KNN) model, a naive Bayes model, a support vector machine (SVM) model, an artificial neural network model, or the like.

An ANN is a statistical learning algorithm modeled from a neural network of a creature. Various neural network models are being researched. Lately, deep neural networks (DNNs) are attracting attention. A DNN is an artificial neural network model including an input layer, an output layer, and several hidden layers between the input layer and the output layer. Like general artificial neural networks, DNNs may model complex nonlinear relationships. Various types of models have been researched for DNNs. For example, there are a convolutional neural network (CNN), a recurrent neural network (RNN), a restricted Boltzmann machine (RBM), a deep belief network (DBN), a generative adversarial network (GAN), a relation network (RL), and the like.

The following description employs a deep learning model that generates a second stained image from an input first stained image. Various types of generative models may be used. However, for convenience of description, the following description will be described on the basis of a GAN which is a generative adversarial model.

A device for analyzing a TSR using a stained image of tissue is referred to as an "analysis device" below. The analysis device is a device that can process data and have a form of a personal computer (PC), a smart device, a server, or the like.

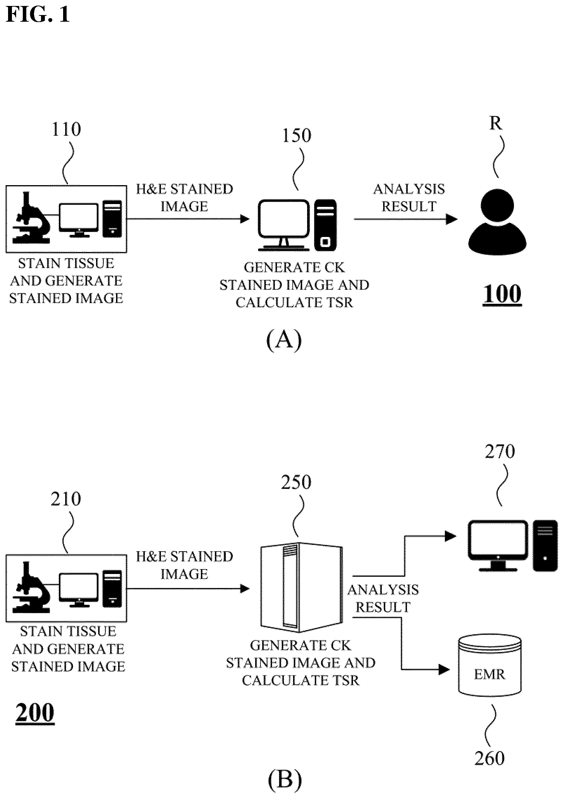

FIG. 1 illustrates an example of a system 100 for predicting a TSR. FIG. 1 shows examples in which the analysis device is a computer terminal 150 and a server 250.

FIG. 1A shows the system 100 for a user to predict a TSR using the computer terminal 150. A stained image generation device 110 is a device that stains a tissue slide and scans the stained tissue slide to generate a stained image. The stained image generation device 110 generates a first stained image (H&E stained image).

The stained image generation device 110 transmits the H&E stained image to the computer terminal 150 through a wired or wireless network. The computer terminal 150 generates a second stained image (CK stained image) on the basis of the H&E stained image. The computer terminal 150 may calculate a TSR using the H&E stained image and the CK stained image of the same tissue. A detailed TSR calculation process will be described below. The computer terminal 150 provides an analysis result to a user R.

In FIG. 1, the stained image generation device 110 and the computer terminal 150 are shown as separate objects. However, the stained image generation device 110 and the computer terminal 150 may be physically implemented as one device or connected devices.

FIG. 1B shows a system 200 in which a user accesses the analysis server 250 via a user terminal 270 to predict a TSR. A stained image generation device 110 is a device that stains a tissue slide and scans the stained tissue slide to generate a stained image. The stained image generation device 110 generates a first stained image (H&E stained image).

The stained image generation device 110 transmits the H&E stained image to the analysis server 250 through a wired or wireless network.

The analysis server 250 generates a second stained image (CK stained image) on the basis of the H&E stained image. The analysis server 250 may calculate a TSR using the H&E stained image and the CK stained image of the same tissue. A detailed TSR calculation process will be described below.

The analysis server 250 provides an analysis result to the user terminal 270. Also, the analysis server 250 may transmit the analysis result to an electronic medical records (EMR) system 260 of a hospital.

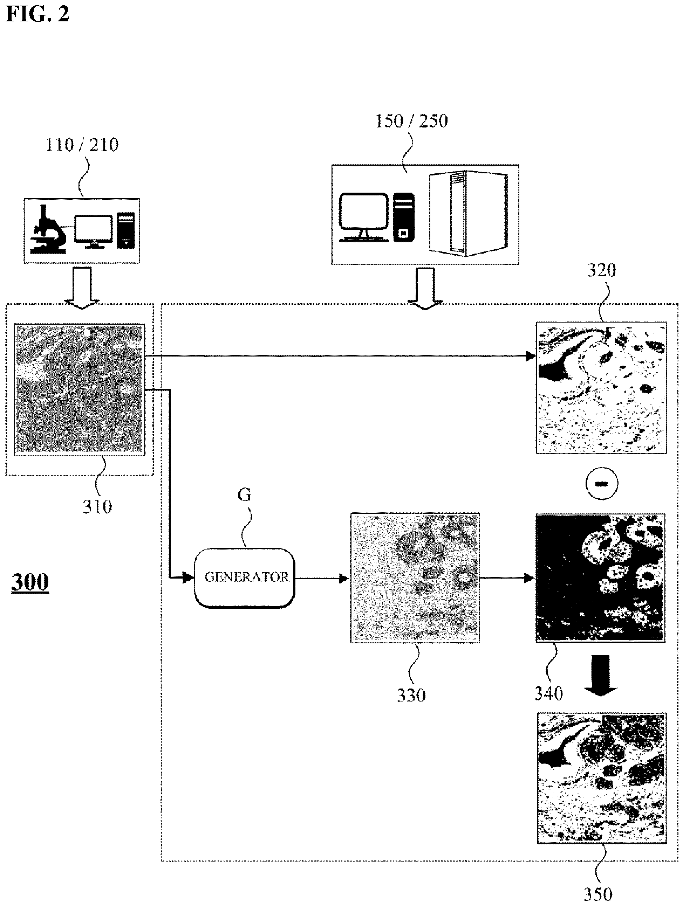

FIG. 2 illustrates an example of a process 300 of predicting a TSR. FIG. 2 shows a schematic example of a TSR prediction process. FIG. 2 shows a process performed by the stained image generation device 110/210 and the analysis device 150/250.

The stained image generation device 110/210 generates an H&E image of specific sample tissue (310).

The analysis device 150/250 receives the H&E image and generates a binary H&E image (320). The analysis device 150/250 generates a CK image on the basis of the received H&E image using a generator G trained in advance (330). The analysis device 150/250 binarizes the CK image (340).

The analysis device 150/250 determines a TSR on the basis of the binary H&E image and the binary CK image. The analysis device 150/250 may subtract the binary CK image from the binary H&E image to calculate a ratio of stroma to the entire area (350).

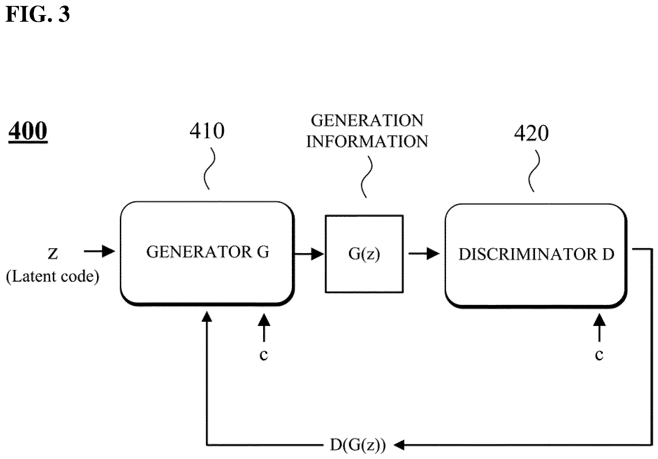

FIG. 3 illustrates an example of a generative adversarial model 400. FIG. 3 is an example of the GAN 400. The GAN corresponds to a non-supervised learning model. The GAN includes a generator G (410) and a discriminator D (420). The GAN is based on a concept that the generator G (410) for generating data and the discriminator D (420) for assessing the generated data learn in opposition to each other to gradually improve performance. Each of the generator G (410) and the discriminator D (420) may be generated using one of various machine learning models. For example, the generator G (410) may be implemented via a model such as U-net, an autoencoder, or the like.

The discriminator D (420) discriminates the generated data as real data or fake data. The generator G is trained to receive a latent code z and generate data, that is, information for deceiving the discriminator D. The generator G generates data G(z), and the discriminator D generates a distinction result of G(z). The generator G has an objective function of minimizing 1-D(G(z)). As a result, when D(G(z)) is 1, the value is minimized, and the generator G is trained so that the discriminator D (420) may mistake G(Z) for the original data.

In a conditional GAN (cGAN), certain condition information c is additionally input to each of the generator G (410) and the discriminator D (420).

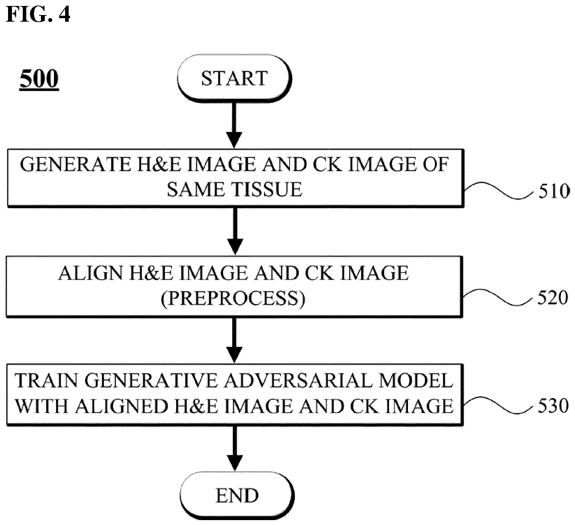

FIG. 4 illustrates an example of a schematic process 500 of training a generative adversarial model.

A developer may carry out a learning process using the above-described analysis device. Alternatively, the developer may carry out a learning process using an additional computer device. The learning process is assumed to be performed by a computer device.

The learning process is coarsely divided into a process of preparing training data and a process of training a generative adversarial model with the training data.

The process of generating training data will be described. Training data includes an H&E image and a CK image of specific tissue. Accordingly, a process is necessary to generate an H&E image and a CK image first (510). The H&E image and the CK image are a pair of images of the same tissue. Training data includes pairs of an H&E image and a CK image of a plurality of samples (i.e., a plurality of pairs of images).

A researcher explains a process of preparing the training data.

369 gastric adenocarcinoma patients were selected for a cohort. The cohort included the patients in clinical stages II to IV. The cohort included the patients who underwent curative surgery at Samsung Seoul Hospital in 2014 and 2015. The patients belonging to the cohort did not undergo neoadjuvant chemotherapy and did not die in 30 days after the surgery. The patients belonging to the cohort had no confirmed distant metastases or other tumors at the time of surgery. Table 1 to be described below includes clinical information of the cohort.

As a slide for staining, a paraffin tissue section prepared after fixation in formalin was cut into 3μm. Each of formalin-fixed paraffin-embedded (FFPE) samples was stained using an automatic H&E staining device. The 369 samples represent the 369 patients. As the samples representing the patients, slides showing the deepest invasion in the tumor were selected, clinical stages of the patients were determined from the slides.

Each of the FFPE samples underwent immunohistochemistry (IHC) staining. The researcher used CK staining. The researcher stained the slides using a Bond-MAX automated IHC stainer (a product of Leica Biosystem corp.) and a commercial reagent (a product of Novocastra corp.). The researcher generated 200-fold magnified images (tissue 0.032μm/pixel) from the CK stained slides using a digital scanner (a product of Aperio Technologies corp.). The CK stained images are referred to as "CK whole slide images (WSIs)."

To perform H&E staining on the same samples again, the researcher washed the CK stained slides with distilled water and then performed H&E staining thereon. The H&E staining process includes a process of incubating the slides in a certain concentration of ethanol and $KMnO_4$ and staining the slides with a staining reagent. Since H&E staining is a general process, detailed description thereof will be omitted. Also, images were generated from the H&E stained slides by the digital scanner. The H&E stained images are referred to as "H&E WSIs."

As described above, the researcher prepared an H&E WSI and a CK WSI of exactly the same tissue section. The researcher confirmed that an H&E WSI and a CK WSI of the same tissue section do not have nonlinear deformations but certain vertical and horizontal shifts occur between the H&E WSI and the CK WSI. Therefore, the researcher aligned a pair of an H&E WSI and a CK WSI of the same section through a certain preprocessing process so that the pair of the H&E WSI and the CK WSI shows the same area as much as possible at a pixel level (520).

The computer device trains a generative adversarial model using aligned pairs of an H&E WSI and a CK WSI (530). In brief, a cGAN receives a latent code and generates a CK WSI. A generative model is trained to receive an H&E WSI and generate a CK WSI. A condition c input to the generative model is information for generating a fine CK WSI. A discrimination model receives a generated (fake) CK WSI and a real CK WSI and distinguish the generated CK WSI. The discrimination model also receives the condition c. When this process is repeated, parameters of the generative model and the discrimination model are optimized, and thus the generative adversarial model is trained.

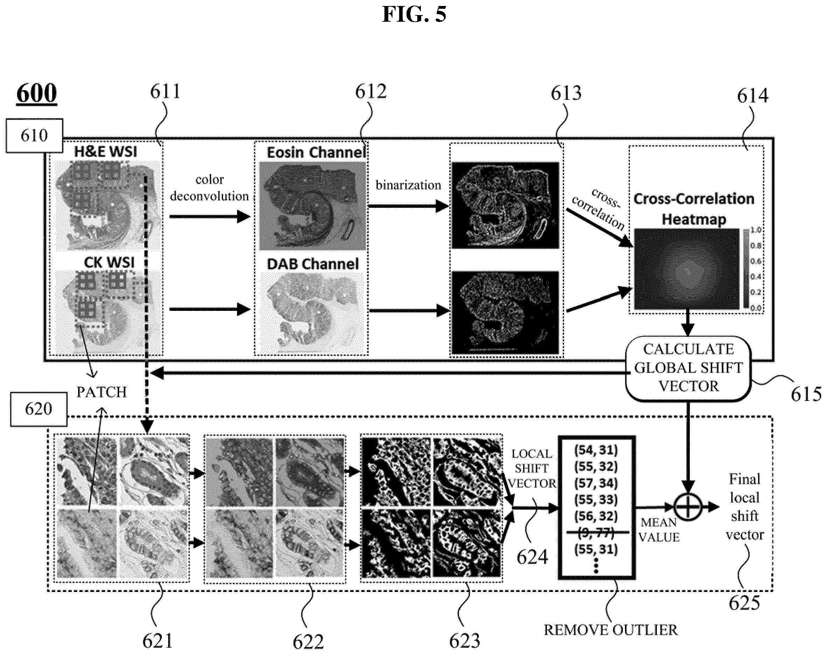

FIG. 5 illustrates an example of a process 600 of aligning an H&E image and a CK image.

An image alignment process includes a global process and a local process. The global process is a process of coarsely aligning a pair of WSIs of the same section by calculating a global shift vector. The local process is a process of precisely aligning the pair of WSIs by calculating a local shift vector for each local area.

A global shift vector calculation process 610 will be described below.

A computer device may down-sample an H&E WSI and a CK WSI at a certain ratio (611). The researcher performed 32-times down-sampling on the H&E WSI and the CK WSI of the same tissue section.

The computer device performs color deconvolution on each of the down-sampled images (612). Here, the color deconvolution converts each of the H&E WSI and the CK WSI from a RGB color space to a hematoxylin-eosin-diaminobenzidine (DAB) (HED) color space. Since CK antibodies mainly stain cytoplasm, an eosin channel may be extracted from the H&E image, and a DAB channel may be extracted from the CK image.

The computer device binarizes the two channel images whose color space has been converted (613). Here, the computer device uses a threshold value which is a criterion for binarization. As the threshold value, an experimentally appropriate value may be used. The threshold value may be set for each small area of an entire image rather than for the entire image. For example, as the threshold value, a value obtained by subtracting a specific offset value from a weighted pixel value average of a neighbor area (block) may be used.

Meanwhile, the computer device may remove ignorable small objects after binarizing the images.

The computer device may generate a two-dimensional (2D) cross-correlation heatmap of the two binary images (614). The cross-correlation may be calculated using a fast Fourier transform (FFT) algorithm. The computer device may determine a global shift vector on the basis of the position of a maximum value in the cross-correlation heatmap (615). In other words, the computer device can acquire global information for aligning one image with another image.

The computer device may align (global alignment) the H&E WSI and the CK WSI on the basis of the global shift vector. For more precise alignment, the computer device may perform local alignment after the global alignment. In some cases, the computer device may determine a local shift vector for each patch and only perform local alignment on each patch without performing global alignment.

A local shift vector calculation process 620 will be described below.

An entire image may be divided into patches having a certain size. One patch may have a certain horizontal×vertical size (pixels). Local alignment is a process of aligning each patch. For example, when an entire image has a size of 10240×10240 pixels, the computer device may perform local alignment on the basis of patches having a size of 1024×1024 pixels. The computer device may align each patch in the same way as in global alignment except for patches having a white background. In other words, the computer device may down-sample an H&E WSI and a CK WSI from each patch (621), perform color deconvolution on the WSIs (622), binarize the WSIs (623), and then calculate local shift vectors on the basis of a maximum value in the cross-correlation heatmap (624). FIG. 5 shows no cross-correlation heatmap of patches.

The computer device may determine a local shift vector for each patch (624). The computer device may calculate a mean value of local shift vectors for a plurality of patches (625). In this process, the computer device may remove a local shift vector having an outlier and then calculate the mean value of the local shift vectors. The computer device may determine a value obtained by adding the mean value of local shift vectors and the global shift vector as a final local shift vector (626).

Meanwhile, a variety of methods may be used to determine a local shift vector. For example, (i) the computer device may calculate a local shift vector for each patch and perform local alignment on the patch. (ii) The computer device may calculate a local shift vector for each patch and calculate a final local shift vector for the patch by adding the global shift vector to the local shift vector. (iii) As described above, the computer device may calculate a local shift vector for each patch, calculate a mean value of the local shift vectors, and then calculate a final local shift vector by adding the mean value to the global shift vector. In this case, the computer device aligns each patch on the basis of the final local shift vector.

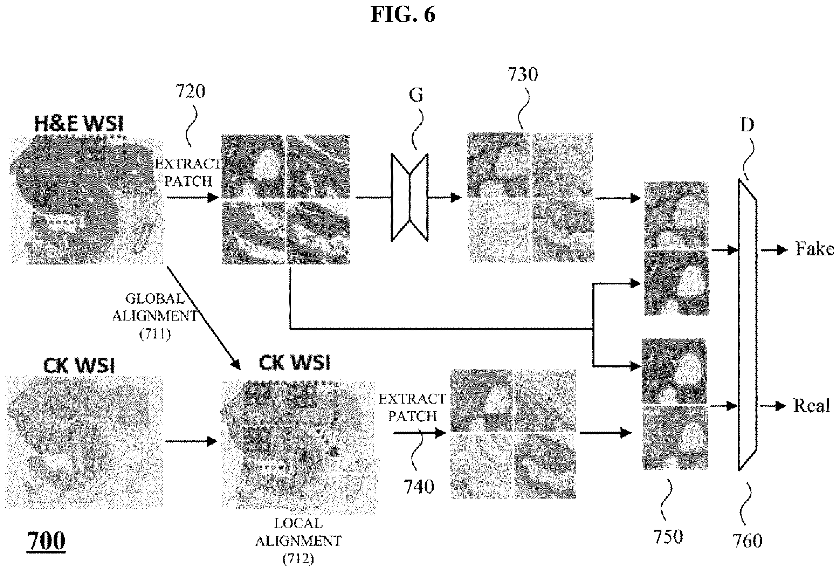

FIG. 6 illustrates an example of a process 700 of training a generative adversarial model using an H&E image and a CK image. FIG. 6 may be a training process after the H&E WSI and the CK WSI are aligned in patch units in FIG. 5. The researcher used a Pix2Pix cGAN among cGAN models. Pix2Pix inputs an edge image of an object to the generator G to generate a target image. This is just an example, and various generative models may be used in generating a CK WSI from an H&E WSI.

Global alignment may be performed on the whole CK WSI on the basis of the whole H&E WSI paired with the CK WSI (711). For each patch of the CK WSI, a final local shift vector may be determined. Each patch of the CK WSI is locally aligned (712). This corresponds to the preprocessing process described in FIG. 5.

The generative adversarial model includes the generator G and the discriminator D. The generator G may use a network having the U-net architecture. The discriminator D may use a PatchGAN for discriminating whether the image generated is real or fake by each patch unit.

Each pair of an H&E WSI and a CK WSI of the same tissue section may be identified as the same patch. The computer device extracts an H&E WSI from each patch (720). The computer device generates a virtual CK patch by inputting each patch of the H&E WSI to the generator G (730). The computer device generates a virtual CK image G(x) by inputting an H&E patch image x to the generator G.

The computer device extracts each patch from the aligned CK WSI (740). The computer device inputs the virtual CK patch and a real CK patch corresponding thereto to the discriminator D (750). The computer device may discriminate by each patch unit with the discriminator (760). A real CK image is assumed to be y. The discriminator D is trained to discriminate between a pair {x, y} and a pair {x, G(x)}. The generator G is trained so that the generated virtual CK image is not distinguished from the real CK image.

The Pix2Pix architecture basically includes a dropout layer in the generator G. The dropout layer causes input noise to be generated as a highly stochastic result. However, a model that generates a CK image requires a predictable and deterministic result. Accordingly, the generator G may not include a dropout layer.

An objective function of the cGAN may be represented as shown in Equation 1 below.

[Equation 1]

$$\mathcal{L}_{cGAN}(G, D) = \mathbb{E}_{x,y}[\log D(x, y)] + \mathbb{E}_x[\log(1 - D(x, G(x)))]$$

The generator G is trained to minimize the objective function, and the discriminator D is inversely trained to maximize to maximize the objective function. The generator G does not only reduce loss of the discriminator but also approximates virtual distribution to real distribution using an L1 loss. Here, it is necessary to consider an L1 loss of the HED color space as well as an L1 loss of the RGB color space. Equation 2 below corresponds to an L1 loss function of the RGB color space, and Equation 3 below corresponds to an L1 loss function of the HED color space.

[Equation 2]

$$\mathcal{L}_{L1}(G)^{RGB} = \mathbb{E}_{x,y}[\|y^{RGB} - G(x)^{RGB}\|_1]$$

[Equation 3]

$$\mathcal{L}_{L1}(G)^{HED} = \mathbb{E}_{x,y}[\|y^{HED} - G(x)^{HED}\|_1]$$

Therefore, a final loss function of the generative adversarial model may be represented as shown in Equation 4 below.

[Equation 4]

$$G^* = \arg \min_G \max_D \mathcal{L}_{cGan}(G, D) + \lambda_1 \mathcal{L}_{L1}(G)^{RGB} + \lambda_2 \mathcal{L}_{L1}(G)^{HED}$$

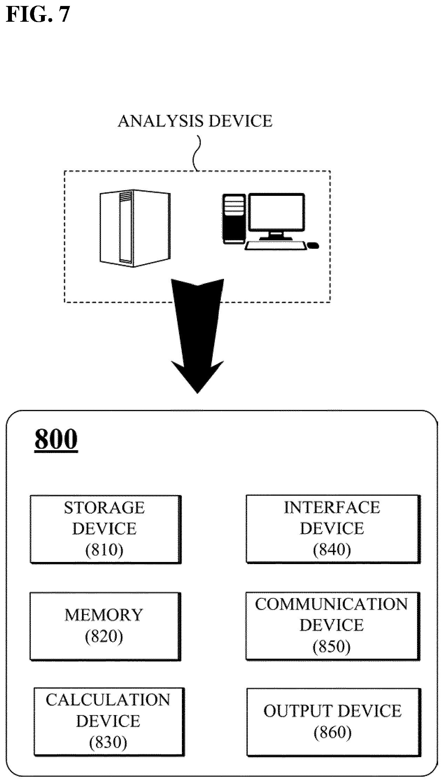

FIG. 7 illustrates an example of an analysis device 800. The analysis device 800 corresponds to the above-described analysis device (150 and 250 of FIG. 1). The analysis device 800 may be physically implemented in various forms. For example, the analysis device 800 may have the form of a computer device, such as a PC, a network server, a data processing chipset, or the like.

The analysis device 800 may include a storage device 810, a memory 820, a calculation device 830, an interface device 840, a communication device 850, and an output device 860.

The storage device 810 may store a first stained image of a specific sample.

The storage device 810 may store a program or code for binarizing an image.

The storage device 810 may store a deep learning model for generating a second stained image on the basis of the first stained image. The deep learning model may be the above-described generative adversarial model.

The storage device 810 may store the second stained image calculated by the deep learning model.

The storage device 810 may store another program or code for image processing.

Also, the storage device 810 may store a command or program code for a process of calculating a TSR as described above.

The storage device 810 may store a TSR which is an analysis result.

The memory 820 may store data, information, and the like generated in a process in which the analysis device 800 calculates the TSR.

The interface device 840 is a device to which a certain command and data are externally input. The interface device 840 may receive the first stained image from an input device or external storage device which is physically connected thereto. The first stained image may be an H&E stained image.

The communication device 850 is a component that receives and transmits certain information through a wired or wireless network. The communication device 850 may receive the first stained image from an external object. Alternatively, the communication device 850 may transmit the analysis result to an external object such as a user terminal.

The interface device 840 and the communication device 850 are components that receive certain information and images from a user or another physical object, and thus may be collectively referred to as an input device. Alternatively, an input device may be an interface in a route for transmitting the first stained image or a request received by the communication device 850 to the inside of the analysis device 800.

The output device 860 is a device that outputs certain information. The output device 860 may output an interface required for a data processing process, the analysis result, and the like.

The calculation device 830 may predict a TSR using the command or program code stored in the storage device 810.

The calculation device 830 may generate the virtual second stained image on the basis of the first stained image using the deep learning model stored in the storage. The virtual second stained image may be a CK stained image.

The calculation device 830 may generate a first binary image by binarizing the first stained image.

The calculation device 830 may generate a second binary image by binarizing the virtual second stained image.

The calculation device 830 may calculate a TSR on the basis of the first binary image and the second binary image.

The calculation device 830 may identify a stroma area in the entire tissue on the basis of a result of removing a white area in the second binary image from a white area in the first binary image. Subsequently, the calculation device 830 may calculate the TSR by calculating a ratio of the stroma area to the entire tissue area.

The calculation device 830 may convert an H&E WSI into a virtual CK WSI using a trained generative model. The calculation device 830 may separately convert patches of the H&E WSI into patches of the CK WSI. The calculation device 830 may determine a tumor area and a stroma area in each patch and finally calculate the TSR for the entire image. Alternatively, the calculation device 830 may generate one H&E WSI and one CK WSI by combining the patches and calculate the TSR on the basis of the H&E WSI and the CK WSI.

The calculation device 830 binarizes the H&E WSI (or the individual patches) and the CK WSI (or the individual patches). The calculation device 830 subtracts (removes) a white area (tumor area) in the binary CK WSI from a white area (stroma area) in the binary H&E WSI. The calculation device 830 may calculate the TSR by determining a ratio of an area from which tumor tissue has been removed to the entire stroma area. In other words, the calculation device 830 may calculate a TSR as shown in Equation 5 below.

[Equation 5]

$$TSR = \frac{stroma\ area}{tumor\ area + stroma\ area} \times 100\%$$

The calculation device 830 may be a device such as a processor which processes data and certain calculation, an application processor (AP), or a chip on which a program is embedded.

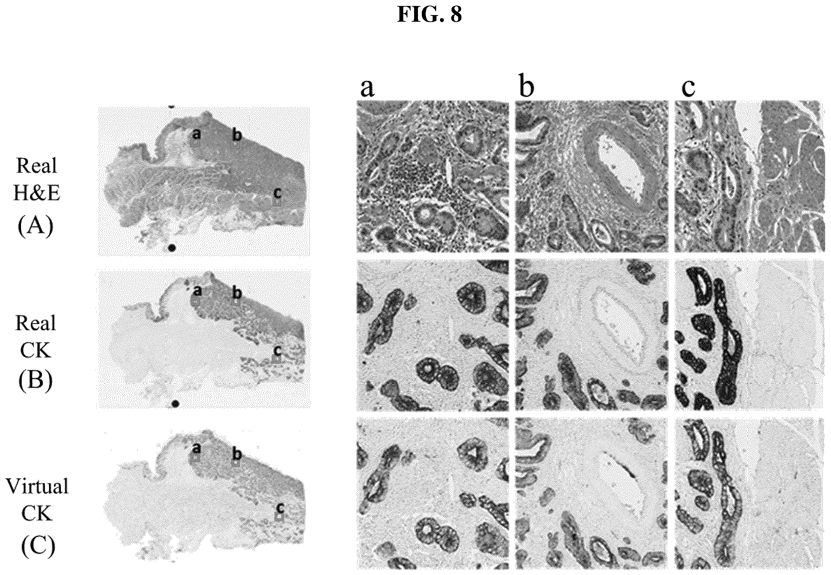

FIG. 8 is an example of a virtual CK image generated by a generative adversarial model. FIG. 8 shows a result of generating a virtual CK image C on the basis of a real H&E image A. Comparing a real CK image B with the virtual CK image C, tumor cells are stained with high accuracy in the virtual CK image.

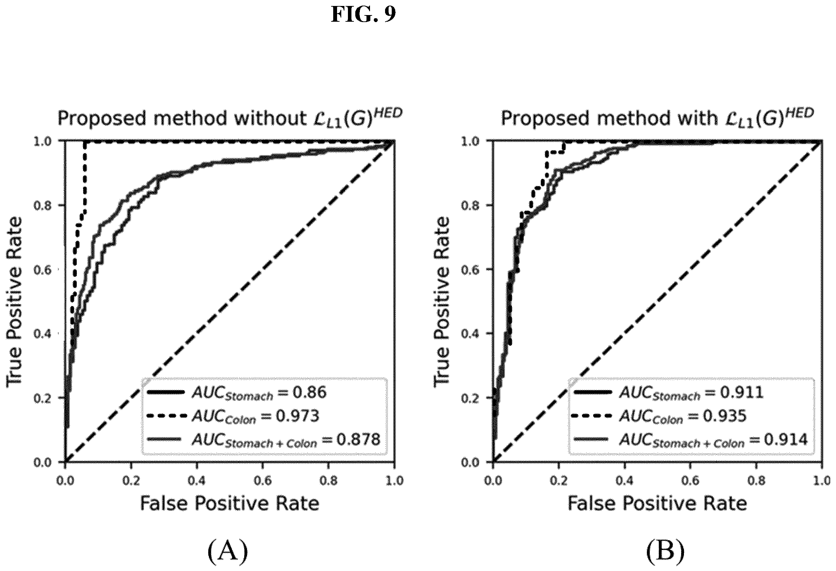

FIG. 9 is an example of evaluating accuracy of TSR prediction results based on a deep learning model. The researcher calculated TSRs of the above-described cohort (the 369 patient samples) using the proposed TSR prediction method. Also, an expert pathologist evaluated the samples. Results based on a generative adversarial model were compared with actual evaluation results of the pathologist. TSRs are measured as ratio values. However, it is difficult to evaluate accuracy of each measured value, and thus evaluations are made as low TSRs and high TSRs. Data used in FIG. 9 corresponds to stomach data, colon data, and stomach + colon data.

FIG. 9A shows results of a model that does not use the loss function (Equation 3) of the HED color space, and FIG. 9B shows results of a model that uses the loss function of the HED color space. In the case of considering the loss function of the HED color space, areas under the curves (AUCs) were high (all were 0.9 or above). Even in the case of not using the loss function of the HED color space, evaluation results were considerably high.

The virtual CK image generation method and TSR calculation method described above may be implemented as a program (or application) including an executable algorithm that may be executed in a computer. The program may be stored and provided in a transitory or non-transitory computer-readable medium.

The non-transitory computer-readable medium is a medium that semi-permanently stores data and is readable by a device, rather than a medium that stores data for a short time period such as a register, a cache, a memory, or the like. Specifically, the various applications or programs described above may be stored and provided in a non-transitory computer-readable medium such as a compact disc (CD), a digital versatile disc (DVD), a hard disk, a Blu-ray disc, a universal serial bus (USB) memory, a memory card, a read-only memory (ROM), a programmable read-only memory (PROM), an erasable PROM (EPROM), an electrically erasable PROM (EEPROM), a flash memory, or the like.

The transitory computer-readable medium is one of various random-access memories (RAMs) such as a static RAM (SRAM), a dynamic RAM (DRAM), a synchronous DRAM (SDRAM), a double data rate (DDR) SDRAM, an enhanced SDRAM (ESDRAM), a synclink DRAM (SLDRAM), and a direct Rambus RAM (DRRAM).

The embodiments and the drawings appended to the present specification clearly describe only a part of the technical spirit of the examples described herein, and it is obvious that all modified examples and specific embodiments that can be easily inferred by those of ordinary skill in the art within the technical spirit included in the specification and drawings of the examples described herein fall in the scope of the present embodiment.

**Claims**

1. A method of predicting a tumor-stroma ratio (TSR) on the basis of a deep learning model, the method comprising:

   receiving, by an analysis device, a first stained image of target tissue;
   generating, by the analysis device, a second stained image by inputting the first stained image into a trained deep learning model;
   generating, by the analysis device, a first binary image of the first stained image;
   generating, by the analysis device, a second binary image of the second stained image; and
   calculating, by the analysis device, a TSR of the target tissue on the basis of the first binary image and the second binary image.

2. The method of claim 1, wherein the first stained image is a hematoxylin & eosin (H&E) stained image, and the second stained image is a cytokeratin (CK) stained image.

3. The method of claim 1, wherein the deep learning model comprises:

a generator configured to receive a real first stained image and generate a virtual second stained image; and a discriminator configured to discriminate whether the second stained image output by the generator is real or not, wherein the discriminator is a patch generative adversarial network (PatchGAN).

4. The method of claim 1, wherein the deep learning model is trained to

divide the first stained image into patches having a certain size, generate patches of the second stained image corresponding to the patches of the first stained image, and discriminate using a pair of patches corresponding to each other among the patches of the first stained image and the patches of the second stained image.

5. The method of claim 1, wherein the deep learning model is trained with training data,

wherein the training data includes a real first stained image and a real second stained image of a same tissue section, and the real first stained image and the real second stained image are data obtained by performing global alignment on the entire images at a pixel level and performing local alignment on a plurality of areas constituting the entire images.

6. The method of claim 5, wherein each of the global alignment and the local alignment comprises:

performing color deconvolution on the two images to be aligned; binarizing the two images having undergone color deconvolution; generating a cross-correlation heatmap of the two binarized images; and calculating a shift vector on the basis of a maximum value in the heatmap.

7. The method of claim 6, wherein the local alignment is performed by aligning each of the plurality of areas on the basis of a sum of a mean value of shift vectors of the plurality of areas and the shift vector value of the global alignment.

8. The method of claim 1, wherein the deep learning model is trained additionally using a loss function of a hematoxylin-eosin-diaminobenzidine (DAB) (HED) color space.

9. An analysis device for predicting a tumor-stroma ratio (TSR) on the basis of a deep learning model, the analysis device comprising:

an input device configured to receive a first stained image of target tissue; a storage device configured to store a generative adversarial model for generating a second stained image on the basis of the first stained image; and a calculation device configured to generate a first binary image by binzarizing the received first stained image, generate a virtual second binary image by inputting the received first stained image to the generative adversarial model, generate a second binary image by binarizing the virtual second stained image, and calculate a TSR of the target tissue on the basis of the first binary image and the second binary image.

10. The analysis device of claim 9, wherein the calculation device calculates a stroma ratio of an entire area by subtracting the second binary image from the first binary image.

11. The analysis device of claim 9, wherein the first stained image is a hematoxylin & eosin (H&E) stained image, and the second stained image is a cytokeratin (CK) stained image.

12. The analysis device of claim 9, wherein the generative adversarial model comprises:

a generator including U-net architecture without dropout layer; and a discriminator discriminating whether each patch is real or not, wherein the discriminator is a patch generative adversarial network (PatchGAN).

13. The analysis device of claim 9, wherein the generative adversarial model is trained with training data,

wherein the training data includes a real first stained image and a real second stained image of a same tissue section, and
the real first stained image and the real second stained image are data obtained by performing global alignment on the entire images at a pixel level and performing local alignment on a plurality of areas constituting the entire images.

14. The analysis device of claim 9, wherein the deep learning model is trained additionally using a loss function of a hematoxylin-eosin-diaminobenzidine (DAB) (HED) color space.

**FIG. 1**

110

150

R

H&E STAINED
IMAGE

ANALYSIS
RESULT

STAIN TISSUE
AND GENERATE
STAINED IMAGE

GENERATE CK
STAINED IMAGE AND
CALCULATE TSR

**100**

(A)

270

250

210

H&E STAINED
IMAGE

ANALYSIS
RESULT

STAIN TISSUE
AND GENERATE
STAINED IMAGE

GENERATE CK
STAINED IMAGE AND
CALCULATE TSR

EMR

**200**

260

(B)

**FIG. 2**

110 / 210

150 / 250

320

310

G

GENERATOR

300

330

340

350

**FIG. 3**

**FIG. 4**

<u>**500**</u>

```
          ┌─────────┐
          │  START  │
          └────┬────┘
               │
               ▼
┌──────────────────────────────────────┐
│ GENERATE H&E IMAGE AND CK IMAGE OF    │──── 510
│           SAME TISSUE                  │
└──────────────────┬───────────────────┘
                   │
                   ▼
┌──────────────────────────────────────┐
│   ALIGN H&E IMAGE AND CK IMAGE        │──── 520
│           (PREPROCESS)                 │
└──────────────────┬───────────────────┘
                   │
                   ▼
┌──────────────────────────────────────┐
│ TRAIN GENERATIVE ADVERSARIAL MODEL     │──── 530
│ WITH ALIGNED H&E IMAGE AND CK IMAGE    │
└──────────────────┬───────────────────┘
                   │
                   ▼
             ┌─────────┐
             │   END   │
             └─────────┘
```

## FIG. 5

EP 4 300 515 A1

FIG. 6

EP 4 300 515 A1

**FIG. 7**

ANALYSIS DEVICE

**800**

| | |
|---|---|
| STORAGE DEVICE (810) | INTERFACE DEVICE (840) |
| MEMORY (820) | COMMUNICATION DEVICE (850) |
| CALCULATION DEVICE (830) | OUTPUT DEVICE (860) |

FIG. 8

Real
H&E
(A)

Real
CK
(B)

Virtual
CK
(C)

a

b

c

EP 4 300 515 A1

**FIG. 9**

Proposed method without $\mathcal{L}_{L1}(G)^{HED}$

$AUC_{Stomach} = 0.86$
$AUC_{Colon} = 0.973$
$AUC_{Stomach+Colon} = 0.878$

True Positive Rate

False Positive Rate

(A)

Proposed method with $\mathcal{L}_{L1}(G)^{HED}$

$AUC_{Stomach} = 0.911$
$AUC_{Colon} = 0.935$
$AUC_{Stomach+Colon} = 0.914$

True Positive Rate

False Positive Rate

(B)

## INTERNATIONAL SEARCH REPORT

| International application No. |
| --- |
| **PCT/KR2021/004727** |

### A. CLASSIFICATION OF SUBJECT MATTER

**G16H 50/50**(2018.01)i; **G16H 30/40**(2018.01)i; **G16H 50/70**(2018.01)i; **G16H 50/20**(2018.01)i; **G06N 20/00**(2019.01)i; **G06N 3/08**(2006.01)i; **G06T 5/00**(2006.01)i; **G01N 33/483**(2006.01)i

According to International Patent Classification (IPC) or to both national classification and IPC

### B. FIELDS SEARCHED

Minimum documentation searched (classification system followed by classification symbols)

G16H 50/50(2018.01); G06K 9/62(2006.01); G06T 7/00(2006.01); G06T 7/11(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 종양-스트로마 비율 예측(tumor-stroma ratio prediction), 염색 이미지 변환 (stained image conversion), 이진화(binarization), 적대적 생성 모델(generative adversarial network), 컬러 디컨볼루션(color deconvolution), 손실 함수(loss function)

### C. DOCUMENTS CONSIDERED TO BE RELEVANT

| Category* | Citation of document, with indication, where appropriate, of the relevant passages | Relevant to claim No. |
| --- | --- | --- |
| A | XU, Zhaoyang et al. GAN-based virtual re-staining: a promising solution for whole slide image analysis. arXiv:1901.04059v1. pp. 1-16, 13 January 2019 [retrived on 21 October 2021]. Retrieved from <https://arxiv.org/pdf/1901.04059.pdf>.<br>See abstract; and pages 10 and 13. | 1-14 |
| A | BURLINGAME, Erik A. et al. SHIFT: speedy histological-to-immunofluorescent translation of a tumor signature enabled by deep learning. Scientific Reports. 2020, vol. 10, Article No. 17507, pp. 1-14.<br>See entire document. | 1-14 |
| A | TSCHUCHNIG, Maximilian E. et al. Generative adversarial networks in digital pathology: a survey on trends and future potential. Patterns. 11 September 2020, vol. 1, no. 6, Article No. 100089, pp. 1-11.<br>See entire document. | 1-14 |
| A | ZHAO, Ke et al. Artificial intelligence quantified tumour-stroma ratio is an independent predictor for overall survival in resectable colorectal cancer. EBioMedicine. 08 October 2020, vol. 61, Article No. 103054, pp. 1-9.<br>See entire document. | 1-14 |

| ✓ Further documents are listed in the continuation of Box C. | ✓ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **23 November 2021** | **23 November 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

<table>
<tr><td colspan="2" align="center">**INTERNATIONAL SEARCH REPORT**</td><td>International application No.<br>**PCT/KR2021/004727**</td></tr>
</table>

**C.     DOCUMENTS CONSIDERED TO BE RELEVANT**

| Category* | Citation of document, with indication, where appropriate, of the relevant passages | Relevant to claim No. |
|---|---|---|
| A | SALVI, Massimo et al. The impact of pre- and post-image processing techniques on deep learning frameworks: a comprehensive review for digital pathology image analysis. Computers in Biology and Medicine. 2021 (online publication date: 21 November 2020), vol. 128, Article No. 104129, pp. 1-24.<br>See entire document. | 1-14 |
| A | US 2020-0258223 A1 (TEMPUS LABS, INC.) 13 August 2020 (2020-08-13)<br>See entire document. | 1-14 |
| A | US 2017-0154420 A1 (VENTANA MEDICAL SYSTEMS, INC. et al.) 01 June 2017 (2017-06-01)<br>See entire document. | 1-14 |

Form PCT/ISA/210 (second sheet) (July 2019)

INTERNATIONAL SEARCH REPORT
Information on patent family members

International application No.

**PCT/KR2021/004727**

| Patent document cited in search report | | | Publication date (day/month/year) | Patent family member(s) | | | Publication date (day/month/year) |
|---|---|---|---|---|---|---|---|
| US | 2020-0258223 | A1 | 13 August 2020 | EP | 3794551 | A1 | 24 March 2021 |
| | | | | US | 10957041 | B2 | 23 March 2021 |
| | | | | US | 2019-0347557 | A1 | 14 November 2019 |
| | | | | WO | 2019-222289 | A1 | 21 November 2019 |
| US | 2017-0154420 | A1 | 01 June 2017 | AU | 2015-265811 | A1 | 20 October 2016 |
| | | | | AU | 2015-265811 | B2 | 30 April 2020 |
| | | | | CA | 2944831 | A1 | 03 December 2015 |
| | | | | CA | 2944831 | C | 31 December 2019 |
| | | | | EP | 3149700 | A1 | 05 April 2017 |
| | | | | EP | 3149700 | B1 | 12 December 2018 |
| | | | | JP | 2017-529513 | A | 05 October 2017 |
| | | | | JP | 6763781 | B2 | 30 September 2020 |
| | | | | US | 10275880 | B2 | 30 April 2019 |
| | | | | WO | 2015-181371 | A1 | 03 December 2015 |

Form PCT/ISA/210 (patent family annex) (July 2019)